# EUROPÄISCHE PATENTANMELDUNG

(11) **EP 3 336 571 A1**
(43) Veröffentlichungstag der Anmeldung: **20.06.2018**
(21) Anmeldenummer: 17164986.6
(22) Anmeldetag: 05.04.2017
(51) Int. Cl.: G01R 33/50, G01R 33/48, G01R 33/561, G01R 33/56

(54) **ZUORDNEN VON MR-FINGERABDRÜCKEN AUF BASIS NEURONALER NETZE**

(71) Anmelder: Siemens Healthcare GmbH, 91052 Erlangen (DE); Friedrich-Alexander-Universität Erlangen-Nürnberg, 91054 Erlangen (DE)
(72) Erfinder: Hoppe, Elisabeth, 91052 Erlangen (DE); Maier, Andreas, 91054 Erlangen (DE); Pfeuffer, Josef, 91358 Kunreuth (DE)

(57) **Zusammenfassung**

Die vorliegende Erfindung betrifft ein Verfahren zum Ermitteln von MR-Parametern (T1, T2), mit den Schritten eines Erfassens (S101) eines MR-Fingerabdrucks eines Voxels mittels einer Pulssequenz; eines Eingebens (S102) des MR-Fingerabdrucks in die Eingabeschicht eines trainierten neuronalen Netzes; und eines Ausgebens (S103) zumindest eines MR-Parameters (T1, T2) zu dem MR-Fingerabdruck an der Ausgabeschicht des neuronalen Netzes.

## Beschreibung

Die vorliegende Erfindung betrifft ein Verfahren zum Ermitteln von MR(Magnetresonanz)-Parametern auf Basis von MR-Fingerabdrücken.

MR-Parameter sind von Bedeutung für Magnetresonanztechniken, beispielsweise für die Magnetresonanztomographie, einem bildgebenden Verfahren zur Darstellung von Körpergewebe. Einige Atomkerne des zu untersuchenden Gewebes besitzen einen Eigendrehimpuls (Kernspin) und sind dadurch magnetisch. Diese Kerne erzeugen nach dem Anlegen eines statischen Magnetfeldes eine longitudinale Magnetisierung in Richtung des statischen Feldes. Durch ein kurzzeitig angelegtes zusätzliches hochfrequentes Wechselfeld im Radiofrequenzbereich (RF) lässt sich die longitudinale Magnetisierung aus der Richtung des statischen Feldes auslenken, also teilweise oder ganz in eine transversale Magnetisierung umwandeln, wodurch in einer Empfängerspule eine elektrische Spannung induziert wird. Nach Abschalten des hochfrequenten Wechselfeldes nimmt die transversale Magnetisierung ab, die Kernspins richten sich also wieder parallel zum statischen Magnetfeld aus. Diese sogenannte Relaxation erfolgt mit einer charakteristischen Abklingzeit. Verschiedene Gewebearten unterscheiden sich in ihren MR-Parametern bzw. in ihren MR-Parameter-Kombinationen, beispielsweise in der longitudinalen Relaxationszeit T1 und/oder in der transversalen Relaxationszeit T2.

Bei einem MR-Fingerprinting (MRF) werden pro Gitterpunkt (Voxel) in einem dreidimensionalen Gitter charakteristische Zeitserien als MR-Signal erhalten. Diese Zeitserien werden als MR-Fingerabdruck des Voxels bezeichnet. Anhand eines Vergleichs mit simulierten Zeitserien, bei denen die MR-Parameter für die Simulation bekannt sind, lassen sich die MR-Parameter des Voxels bestimmen.

Das Verfahren zum Erhalten dieser Zeitserien ist beispielsweise in Jiang Y, Ma D, Seiberlich N, Gulani V, Griswold MA, "MR Fingerprinting Using Fast Imaging with Steady State Precession (FISP) with Spiral Readout", Magnetic Resonance in Medicine 74:1621-1631 (2015) beschrieben. In dem dort beschriebenen Verfahren werden die MR-Signale mit einer MRF-FISP-Pulssequenz, bei der auf einen adiabatischen Inversionspuls eine Serie von FISP-Aufnahmen folgt, gemessen. In der MRF-FISP-Pulssequenz wird eine sinusförmige Verteilung von Einstellungswinkeln und Wiederholungszeiten in einem Perlin-Rauschmuster verwendet. Ein Verschachteln (Interleave) einer Spiraltrajektorie einer variablen Dichte wird in jeder Wiederholung verwendet. Die Spiraltrajektorie ist nullmomentkompensiert. Es erfordert 24 Verschachtelungen um das Zentrum des k-Raumes abzutasten und 48 Verschachtelungen für einen Bereich von 256 x 256. Es sind auch andere Aufnahmetechniken, beispielsweise kartesische oder radiale, zur Messung der MR-Signale möglich. Die zeitliche Abfolge von zu schaltenden Gradienten, einzustrahlenden RF-Pulsen und Signal-Aufnahmezeitfenstern einer Aufnahmetechnik wird durch die jeweils zugehörige Pulssequenz vorgegeben.

Um die Eigenschaften des Gewebes, wie beispielsweise die T1- und T2-Relaxationszeiten zu erhalten, wird in der bildgebenden MRF-Technik die gemessene Zeitserie, d.h. der MR-Fingerabdruck des Gewebes, mit einem zuvor simulierten MR-Fingerabdruck aus einem Verzeichnis (Dictionary) mittels einer Mustererkennung (Pattern Recognition) abgeglichen (Matching). Das Verzeichnis mit den MR-Fingerabdrücken wird zuvor konstruiert und umfasst die simulierten Fingerabdrücke für mögliche Kombinationen aller MR-Parameter. Das Verzeichnis wird unter Verwendung der Bloch-Gleichungen und der gleichen Aufnahmeparameter (Acquisition Parameter) wie für die Messung erzeugt, wie beispielsweise einer Verteilung von Einstellwinkeln (Flip Angle), einer Phase der RF-Pulse oder von Wiederholungszeiten.

Durch das Abgleichen wird diejenige simulierte Zeitserie aus dem Verzeichnis ausgewählt, die der gemessenen Zeitserie am besten entspricht. Da für die simulierte Zeitserie aus dem Verzeichnis die T1- und T2-Relaxationszeiten bekannt sind, können diese Parameter auch dem Gewebe aus dem entsprechenden Voxel zugeordnet werden.

Die bekannten Verfahren für eine Mustererkennung zum Abgleichen der gemessenen Fingerabdrücke mit den vorgespeicherten Fingerabdrücken können wie folgt beschrieben werden:
1) Ma D, Gulani V, Seiberlich N, Liu K, Sunshine JL, Duerk JL, Griswold MA, "Magnetic Resonance Fingerprinting", Nature 2013; 495: 187-192, verwenden einen Schablonenabgleich (Template Matching). Hierzu wird ein Vektorskalarprodukt zwischen der gemessenen Zeitserie und allen einzelnen Zeitserien des Verzeichnisses berechnet. Die Parameter derjenigen Zeitserie mit dem größten Skalarprodukt werden ausgewählt und den entsprechenden Voxel zugeordnet.
2) Cauley SF, Setsompop K, Ma D, Jiang Y, Ye H, Adalsteinsson E, Griswold MA, Wald LL, "Fast Group Matching for MR Fingerprinting Reconstruction", Magn. Reson. Med. 2015 Aug; 74(2):523-528 schlagen ein Fast-Group-Matching-Verfahren vor, um das Abgleichen zu beschleunigen. Dies wird nicht durch Vergleichen einer gemessenen Zeitserie mit dem gesamten Verzeichnis erreicht. Stattdessen wird ein Gruppieren ähnlicher Zeitserien durchgeführt. Auf diese Wiese werden M Zeitserien aus dem Verzeichnis auf N Gruppen verteilt. Jede Gruppe weist M/N Elemente auf.

Das Gruppieren wird durchgeführt, indem zunächst ein Anfangssignal S₀ gewählt wird. Das Anfangssignal S₀ wird mit allen anderen Zeitserien des Verzeichnisses verglichen. Die am meisten mit dem Anfangssignal korrelierten M/N Signale werden verwendet, um die erste Gruppe zu erzeugen. Ein neues Signal S₁ wird erzeugt, d.h. ein Durchschnittssignal der ersten Gruppe, das von nun an die Einträge in der ersten Gruppe am besten darstellt. Eine kleinere Gruppenebene einer Hauptkomponentenanalyse (PCA - Principal Component Analysis) wird unter Verwendung einer Singulärwertzerlegung berechnet. Diese Schritte werden wiederholt bis alle Verzeichniselemente verteilt sind. Das Abgleichen einer erfassten Zeitserie mit diesem komprimierten Verzeichnis wird durchgeführt, indem die Zeitserie gegen alle repräsentativen Gruppensignale S₁, ..., S_{N} abgeglichen (gematched) wird. Die Gruppen mit den höchsten Übereinstimmungen werden ausgewählt. Die verbleibenden Gruppen werden anschließend durch eine PCA-Projektion evaluiert. Das beste Ergebnis und seine Parameter werden dann für das Voxel ausgewählt.

Es ist die technische Aufgabe der vorliegenden Erfindung, das Ermitteln von MR-Parametern auf Basis von MR-Fingerabdrücken zu verbessern und zu beschleunigen.

Diese Aufgabe wird durch Gegenstände nach den unabhängigen Ansprüchen gelöst. Vorteilhafte Ausführungsformen sind Gegenstand der abhängigen Ansprüche, der Beschreibung und der Figuren.

Gemäß einem ersten Aspekt wird die Aufgabe durch ein Verfahren zum Ermitteln von MR-Parametern gelöst, mit den Schritten eines Erfassens eines MR-Fingerabdrucks eines Voxels mittels einer Pulssequenz; eines Eingebens des MR-Fingerabdrucks in die Eingabeschicht eines trainierten neuronalen Netzes; und eines Ausgebens zumindest eines MR-Parameters zu dem MR-Fingerabdruck an der Ausgabeschicht des trainierten neuronalen Netzes.

Durch das Verfahren wird der technische Vorteil erreicht, dass eine Geschwindigkeit der Quantifizierung erhöht wird und kein Verzeichnis mehr für den Schritt einer Quantifizierung der MR-Parameter benötigt wird, dessen Speicherplatzbedarf mit den darin enthaltenen möglichen Parameterkombinationen wächst. Das Verzeichnis wird implizit durch das trainierte neuronale Netz repräsentiert, aber - im Vergleich zum Verzeichnis - weist dieses eine konstante Speichergröße auf. Mit dem erfindungsgemäßen Verfahren wird somit wesentlich weniger Speicherplatz benötigt, da der bisher benötigte Speicherplatz für das Wörterbuch (für die Referenzsignale) entfällt.

Grundsätzlich werden bei der MR-Bildgebung unterschiedliche Hochfrequenz-Signale über einen Zeitraum gesammelt, um eine Signalentwicklung für das Volumen zu identifizieren. Eine Ortsauflösung (zur Zuordnung der erfassten Signale zu einem Voxel) wird mittels linear ortsabhängiger Gradientenfelder erreicht. Da unterschiedliche Gewebetypen (z.B. Tumorgewebe) unterschiedliche HF-Signale erzeugen können, werden im Stand der Technik zur Charakterisierung des Gewebetyps häufig Verfahren zur Mustererkennung (Pattern Recognition) im Signalzeitverlauf verwendet.

Die Grundidee, die dem MR-Fingerprinting (MRF) zugrunde liegt, besteht darin, dass mit einem geeigneten Mess- und Rekonstruktionsschema eindeutige Signalentwicklungen - sogenannte Fingerabdrücke - für verschiedene Gewebereiche und/oder Gewebearten erzeugt werden können. Ein MR-Fingerabdruck beinhaltet das Anlegen einer Reihe von variierten Pulssequenzblöcken, die eine bestimmte Signalentwicklungssignatur, d.h. den MR-Fingerabdruck, erzeugen, die für eine bestimmte Kombination von Parametern und Resonanzarten in einem Volumen spezifisch ist. Die Verarbeitung der empfangenen MRF-Signale beinhaltet nicht mehr eine konventionelle Rekonstruktion, sondern den Abgleich mit Referenz-Signalen.

Im Stand der Technik kann die Verarbeitung nach der MR-Messung einen Mustererkennungsalgorithmus umfassen, um die gesammelten Fingerabdrücke mit vorhergesagten Signalentwicklungen aus einem vordefinierten Wörterbuch abzugleichen, was einen erheblichen Rechen- und Speicheraufwand erfordert.

Anstelle des Mustererkennungsalgorithmus wird in dem hier vorgeschlagenen Verfahren eine alternative Methode verwendet, bei der auf die Verwendung eines neuronalen Netzes zur Bestimmung der MR-Parameter zurückgegriffen wird.

Neuronale Netze werden bereits im Bereich der MR-Technologie verwendet. So wird beispielsweise in der Patentanmeldung US 14/682,220 beschrieben, ein neuronales Netz zum Erzeugen von Pulssequenzen zu verwenden, jedoch nicht zum Klassifizieren von gemessenen MR-Fingerabdrücken.

In einer technisch vorteilhaften Ausführungsform des Verfahrens ist die Pulssequenz eine MRF-FISP-Pulssequenz. Dadurch wird beispielsweise der technische Vorteil erreicht, dass sich MR-Fingerabdrücke auf effiziente Weise erhalten lassen.

In einer weiteren technisch vorteilhaften Ausführungsform des Verfahrens umfasst der zumindest eine MR-Parameter eine T1-Relaxationszeit und/oder eine T2-Relaxationszeit. Dadurch wird beispielsweise der technische Vorteil erreicht, dass sich die Relaxationszeiten des Voxels auf schnelle Weise bestimmen lassen.

In einer weiteren technisch vorteilhaften Ausführungsform des Verfahrens werden mehrere MR-Fingerabdrücke benachbarter Voxel in die Eingabeschicht eingegeben und zumindest ein gemeinsamer MR-Parameter für die mehreren MR-Fingerabdrücke an der Ausgabeschicht ausgegeben. Dadurch wird beispielsweise der technische Vorteil erreicht, dass eine stabilere Schätzung oder eine Fehlerabschätzung erhalten wird.

In einer weiteren technisch vorteilhaften Ausführungsform des Verfahrens wird ein einziger MR-Fingerabdruck eines Voxels in die Eingabeschicht eingegeben und mehrere MR-Parameter für benachbarte Voxel werden an der Ausgabeschicht ausgegeben. Dadurch wird beispielsweise der technische Vorteil erreicht, dass eine Auflösung verbessert werden kann.

In einer weiteren technisch vorteilhaften Ausführungsform des Verfahrens wird ein Kontrast zwischen zwei MR-Fingerabdrücken mittels des neuronalen Netzes ausgewertet. Dadurch wird beispielsweise der technische Vorteil erreicht, dass eine Aufnahmesequenz so verändert werden kann, dass der Kontrast maximal wird.

In einer weiteren technisch vorteilhaften Ausführungsform des Verfahrens wird das Verfahren für eine Vielzahl benachbarter Voxel angewendet. Dadurch wird beispielsweise der technische Vorteil erreicht, dass sich Karten und Bilder der entsprechenden MR-Parameter ermitteln lassen.

Gemäß einem zweiten Aspekt wird die Aufgabe durch ein Computerprogramm mit Programmabschnitten zum Durchführen der Verfahrensschritte nach dem ersten Aspekt gelöst, wenn das Computerprogramm auf einem Computer ausgeführt wird.

Gemäß einem dritten Aspekt wird die Aufgabe durch einen elektronisch lesbaren Datenspeicher mit einem Computerprogramm nach dem zweiten Aspekt gelöst.

Gemäß einem vierten Aspekt wird die Aufgabe durch ein neuronales Netz gelöst, mit einer Eingabeschicht zum Eingeben eines MR-Fingerabdrucks eines Voxels; mehreren verdeckten Schichten zum Verarbeiten des MR-Fingerabdrucks; und einer Ausgabeschicht zum Ausgeben zumindest eines MR-Parameters zu dem MR-Fingerabdruck des Voxels. Dadurch werden die gleichen technischen Vorteile wie durch das Verfahren nach dem ersten Aspekt erreicht.

Gemäß einem fünften Aspekt wird die Aufgabe durch ein Magnetresonanzgerät zum Ermitteln von MR-Parametern gelöst, mit einer Erfassungseinheit zum Erfassen eines MR-Fingerabdrucks eines Voxels mittels einer Pulssequenz; und einer Bestimmungseinheit zum Bestimmen zumindest eines MR-Parameters durch Eingeben des MR-Fingerabdrucks in die Eingabeschicht eines trainierten neuronalen Netzes; und Ausgeben des zumindest einen MR-Parameters zu dem MR-Fingerabdruck des Voxels an der Ausgabeschicht des trainierten neuronalen Netzes. Dadurch werden die gleichen technischen Vorteile wie durch das Verfahren nach dem ersten Aspekt erreicht. Als das trainierte neuronale Netz kann in der Bestimmungseinheit ein neuronales Netz gemäß dem vierten Aspekt vorgesehen sein, das trainiert ist.

In einer technisch vorteilhaften Ausführungsform des Magnetresonanzgeräts ist das Magnetresonanzgerät ausgebildet, MR-Parameter einer Vielzahl benachbarter Voxel zu ermitteln. Dadurch wird beispielsweise der technische Vorteil erreicht, dass sich Karten und Bilder der entsprechenden MR-Parameter ermitteln lassen.

Ausführungsbeispiele der Erfindung sind in den Zeichnungen dargestellt und werden im Folgenden näher beschrieben.

Es zeigen:
- Fig. 1: eine schematische Ansicht eines neuronalen Netzes und ein Blockdiagramm eines Verfahrens zum Bestimmen von MR-Parametern;
- Fig. 2: ein Blockdiagramm zum Anlernen des neuronalen Netzes;
- Fig. 3: ein Blockdiagramm für eine Auflösungsvergrößerung;
- Fig. 4: ein Blockdiagramm für eine Berücksichtigung räumlicher Nachbarn;
- Fig. 5: ein Blockdiagramm zum Erzeugen einer Aufnahmesequenz, Messen von Zeitserien und Trainieren des neuronalen Netzes; und
- Fig. 6: eine schematische Darstellung eines Magnetresonanzgeräts zum Ermitteln von MR-Parametern auf Basis von MR-Fingerabdrücken.

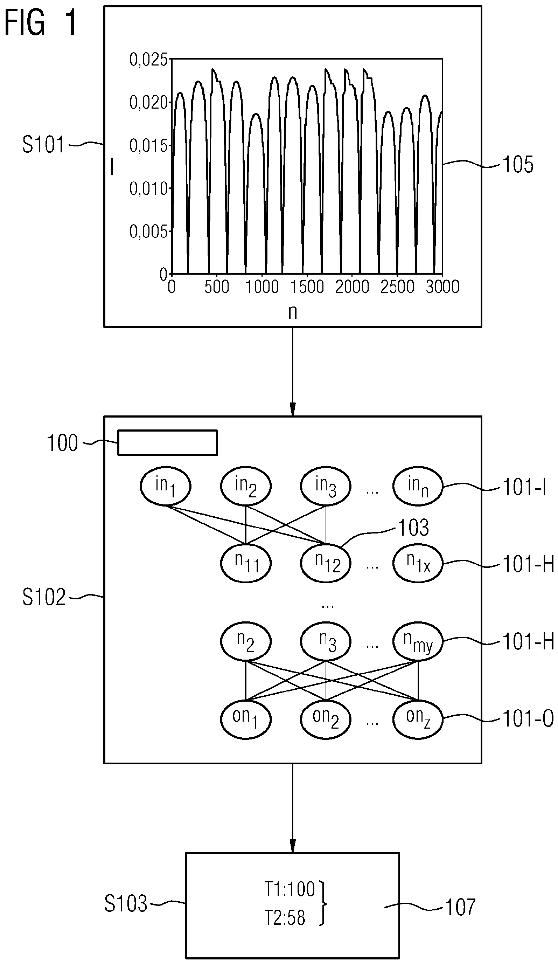

Fig. 1 zeigt eine schematische Ansicht eines neuronalen Netzes 100. Tiefgehendes Lernen (Deep Learning) bezeichnet einen Bereich maschinellen Lernens und eine Klasse von Optimierungsmethoden mithilfe künstlicher neuronaler Netze 100, die zahlreiche Zwischenlagen 101-H zwischen einer Eingabeschicht 101-1 und einer Ausgabeschicht 101-O haben und eine umfangreiche innere Struktur aufweisen. Das Netz 100 ermöglicht auch bei zahlreichen Zwischenlagen 101-H einen stabilen Lernerfolg.

Das Netz 100 kann als künstliches neuronales Netz 100 (ANN - Artificial Neural Network) mit mehreren verdeckten Schichten (Hidden Layer) 101-H beschrieben werden. Die Schichten 101-H werden zum Lernen abstrakter Merkmale aus den Eingabedaten verwendet. Durch die hierarchische Darstellung werden zunächst Merkmale auf niedrigeren Ebenen und danach Merkmale auf höheren Ebenen gelernt.

Das künstliche neuronale Netz 100 basiert auf einer Informationsverarbeitung des Gehirns. Es umfasst ein Netz von Berechnungseinheiten, die Knoten (Nodes) 103 genannt werden. Die Knoten 103 sind miteinander verbunden und mit den Neuronen und Axonen im menschlichen Gehirn vergleichbar. Jeder Knoten 103 erhält Eingabedaten (entweder als tatsächliche Eingabedaten oder als Ausgabedaten eines anderen Knotens 103), führt eine Berechnung auf Basis dieser Eingabedaten durch und leitet das Ergebnis an verbundene Knoten weiter. Die Verbindungen zwischen den Knoten 103 weisen Gewichte auf, die definieren, wie stark die Verbindung zwischen den jeweiligen Knoten 103 ist.

Ein derartiges Netz 100 kann mehrere Schichten 101 aufweisen, wie beispielsweise Convolution-Layer, Pooling-Layer und/oder Fully-connected Layer. Bei dem Convolution-Layer sind nicht alle Neuronen mit den Neuronen der vorherigen Schicht verbunden, sondern es werden Filterkerne angelernt. Mit den Filterkernen und den Eingabedaten wird eine Faltung (Konvolution) durchgeführt und das Ergebnis der Faltung an die anschließende Aktivierungsfunktion weitergeleitet. Beim Pooling-Layer wird die Auflösung verringert und beispielsweise die relevantesten Signale einer Umgebung (z.B. 2*2) beibehalten (Max-Pooling). In der Fully-connected-Layer sind jeweils alle Ausgabeneuronen der vorherigen Schicht mit allen Neuronen der Fully-Connected Schicht verbunden.

Nach den Schichten 101 wird der Ausgabewert an eine Aktivierungsfunktion übergeben. Die Eingabeschicht 101-1 mit den Eingabeknoten In₁, ..., Inₙ nimmt die Daten an, während die Ausgabeschicht 101-O mit den Ausgabeknoten On₁, ..., Onₙ als letzte Schicht die Endausgabe des Netzes 100 bereithält. Zwischen der Eingabeschicht 101-1 und der Ausgabeschicht 101-O können mehrere verdeckte Schichten 101-H mit den Knoten n₁₁, ..., n_{mf} angeordnet sein.

Bevor ein künstliches neuronale Netz 100 zur Berechnung von Ausgabewerten aus Eingabedaten verwendet werden kann, muss dieses trainiert werden, um die Gewichte der Verbindungen zwischen den Knoten 103 der Schichten 101 und die Werte in den Filtern der Convolutional Layer zu lernen. Während einem Trainieren des künstlichen neuronalen Netzes 100 werden Trainingsdaten durch das Netz 100 weitergeleitet und die Ergebnisse werden mit erwarteten Grundwahrheitswerten verglichen. Ein bekannter Lernalgorithmus ist die Back-Propagation. Danach wird der Fehler zwischen den Ergebnissen und den erwarteten Werten berechnet und der Gradient der Fehlerfunktion wird verwendet, um iterativ die Gewichte in dem künstlichen neuronalen Netz 100 zu verändern und den Fehler zu minimieren.

Um ein Abgleichen der MRF-Mustererkennung der erfassten Zeitserie mit denjenigen Zeitserien zu ersetzen, die in dem Verzeichnis umfasst sind, wird stattdessen ein tiefgehendes Lernverfahren auf der Basis des künstlichen neuronalen Netzes 100 verwendet. Das künstliche neuronale Netz 100 wird verwendet, um die Merkmale einer MR-Zeitserie, d.h. eines MR-Fingerabdrucks, zu lernen. Das Netz 100 wird anschließend verwendet, um die quantitativen MR-Parameter, wie beispielsweise die T1- und/oder T2-Ralaxationszeiten, direkt aus dem eingegebenen MR-Fingerabdruck eines Voxels zu bestimmen.

In Schritt S101 wird zunächst der MR-Fingerabdruck 105 eines Voxels mittels einer Pulssequenz erfasst. Der Fingerabdruck wird beispielsweise durch eine Auftragung der normalisierten Signalintensität I gegenüber der Anzahl der Datenpunkte n gebildet. Die Datenpunkte geben hierbei den Zeitverlauf des MR-Signals bei Verwendung der Pulssequenz wieder.

In Schritt S102 wird der MR-Fingerabdruck 105 des Voxels in die Eingabeschicht 101-1 des trainierten neuronalen Netzes 100 eingegeben. Zu diesem Zweck umfasst die Eingabeschicht 101-1 eine Anzahl von n Eingabeknoten In₁, ..., Inₙ. Anschließend wird MR-Fingerabdruck 105 durch das trainierte Netz 100 geleitet. In Schritt S103 wird zumindest ein MR-Parameter, z.B. T1, T2, des Voxels 107 zu dem MR-Fingerabdruck 105 an der Ausgabeschicht 101-O des neuronalen Netzes 100 ausgegeben.

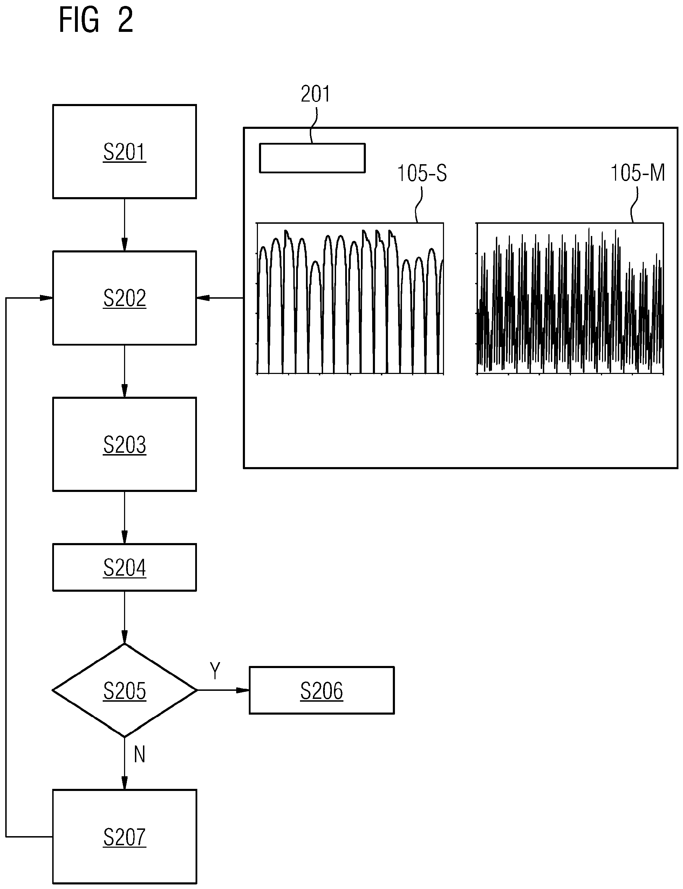

Fig. 2 zeigt ein Blockdiagramm zum Anlernen des neuronalen Netzes 100. Für die Architektur des Systems wird ein künstliches neuronales Netz 100 für ein Regressionsproblem entworfen und angelernt. Bei einem Regressionsproblem sind die Ausgaben des Netzes 100 kontinuierliche Zahlen, während bei einem Klassifikationsproblem die Ausgabe die Wahrscheinlichkeit für eine Klasse oder mehrere Wahrscheinlichkeiten für jeweils mehrere Klassen ist/sind.

Dieses Netz 100 lernt die Merkmale aus den MR-Zeitserien, d.h. MR-Fingerabdrücke, unterschiedlicher Gewebe, beispielsweise unterschiedliche T1- und T2-Kombinationen, mithilfe eines Trainingsprozesses und gibt die zugehörigen quantitativen MR-Parameter aus, wie beispielsweise T1- oder T2-Relaxationszeiten. Die Zeitserien verschiedener T1- und T2-Kombinationen unterscheiden sich jeweils. Das Netz 100 weist Faltungsschichten (Convolutional Layers), vollständig verbundene Schichten (Fully Connected Layers) und nichtlineare Aktivierungen auf. Die Eingabe in das neuronale Netz 100 ist eine gemessene Zeitserie der normalisierten Signalintensität eines Voxels, d.h. der MR-Fingerabdruck des Voxels, und die Ausgabe des neuronale Netzes 100 sind die quantitativen MR-Parameter.

Das Anlernen (Training) des künstlichen neuronalen Netzes 100 wird mit simulierten MR-Zeitserien, d.h. MR-Fingerabdrücken, als Eingabe und den zugehörigen MR-Parametern, mit denen die Simulation durchgeführt worden ist, als Grundwahrheitswerte (Ground Truth Values) durchgeführt. Die Daten werden in Trainings-, Validierungs- und Testdatensätze aufgeteilt.

Validierungsdatensätze werden verwendet, um die Trainingsergebnisse durch ein Verändern von Hyperparametern des Netzes 100 zu verbessern. Die Hyperparameter umfassen beispielsweis die Anzahl verdeckter Schichten im Netz, eine Lernrate, eine Größe und Anzahl der Filterkerne, eine Optimierungsmethode und/oder eine Anzahl an Neuronen pro Schicht. Um das Netz 100 nach einem beendeten Training mit simulierten Daten stabil hinsichtlich eines Artefaktverhaltens, wie beispielsweise einem Rauschen oder Undersampling-Artefakten, zu machen, wird ein Training mit gemessenen Phantomdaten mit bekannten Grundwahrheitswerten, wie beispielsweise T1- und T2-Relaxationszeiten, zur Feinabstimmung der Gewichte des Netzes 100 verwendet. Die Grundwahrheitswerte kommen beispielsweise aus der Anwendung der beschriebenen Matching-Verfahren auf die gemessenen Signale oder sind bereits vorhandene Grundwahrheitswerte, wie z.B. bei einem NIST-Phantom.

In Schritt S201 werden die Gewichte des neuronalen Netzes 100 initialisiert. Hierzu erfolgt beispielsweise eine normale Initialisierung mit Zufallszahlen aus einer Gaußverteilung bei einem Training oder eine Feinabstimmung aus einem gespeicherten Model.

In Schritt S202 werden Eingabedaten 201 zugeführt, die beispielsweise simulierte MR-Fingerabdrücke 105-S und gemessene MR-Fingerabdrücke 105-M umfassen. Nach einer Initialisierung der Gewichte werden die Eingabedaten 201 als Trainingsdatensätze durch das Netz in Vorwärtsrichtung hindurchgeleitet. Bei einem Training des neuronalen Netzes 100 werden die simulierten MR-Fingerabdrücke 105-S verwendet. Bei einer Feinabstimmung werden die gemessene MR-Fingerabdrücke 105-M verwendet.

In Schritt S203 werden die Ergebnisse mit den Grundwahrheitswerten aus dem Verzeichnis verglichen. Bei einem Training werden die MR-Parameter verwendet, die den simulierten MR-Fingerabdrücken 105-S zugeordnet sind. Bei einer Feinabstimmung werden die Phantomparameter der gemessenen MR-Fingerabdrücke verwendet.

In Schritt S204 werden die Fehler zwischen der entsprechenden Ausgabe des neuronalen Netzes 100 und den Grundwahrheitswerten berechnet.

In Schritt S205 wird überprüft, ob die vorgegebene Anzahl an Iterationen erreicht ist oder der Fehler unter einer vorgegebenen Grenze liegt. Falls dies der Fall ist, wird in Schritt S206 das Modell des neuronalen Netzes gespeichert.

Falls dies nicht der Fall ist, werden in Schritt S207 die Gewichte der Knoten 103 in dem Netz 100 mittels einer Rück-Propagation (Back Propagation) und eines Gradienten der Fehlerfunktion aktualisiert.

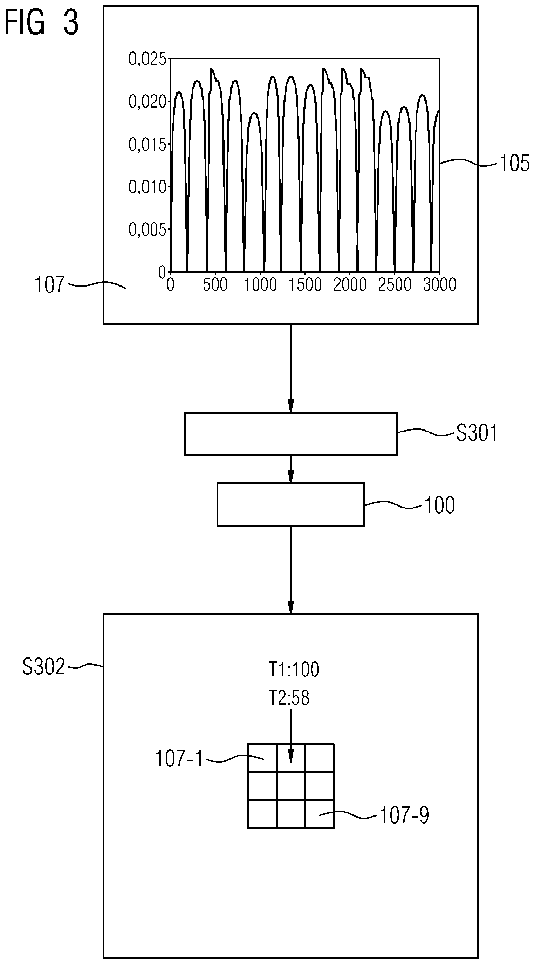

Fig. 3 zeigt ein Blockdiagramm für eine Auflösungsvergrößerung. In Schritt S301 wird ein MR-Fingerabdruck 105 eines einzigen Voxels 107 in das neuronale Netz 100 eingegeben. In Schritt S302 werden anschließend z MR-Parameter für mehrere Voxel 107, ..., 107-9 durch das Netz 100 ausgegeben.

Das Netz 100 kann trainiert werden, um einen partiellen Volumeneffekt (Partial Volume Effect) durch ein Upsampling der Auflösung des MR-Bildes oder der quantitativen Karten zu verbessern (Super-Resolution). Dies kann durch ein Trainieren des Netzes 100 erreicht werden, so dass dieses beispielsweise eine 3x3-Umgebung von Voxeln mit eigenen Parameterwerten, die entsprechend kleinere Abmessungen aufweisen, für einen eingegebenen MR-Fingerabdruck 105 eines einzigen Voxels 107 ausgibt.

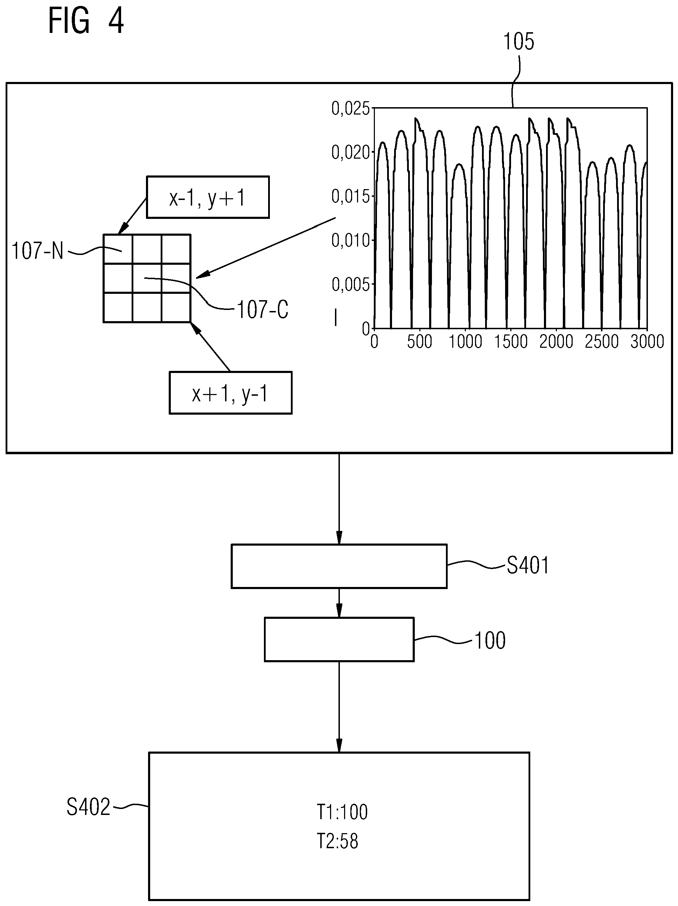

Fig. 4 zeigt ein Blockdiagramm für eine Berücksichtigung räumlicher Nachbarn. Das Netz 100 kann verwendet werden, um ebenfalls den räumlichen Kontext eines Voxels 107 zu berücksichtigen.

In Schritt S401 werden beispielsweise die MR-Fingerabdrücke 105 eines zentralen Voxels 107-C und der benachbarten Voxel 107-N in das Netz 100 eingegeben. Um die Vorhersage der quantitativen MR-Parameter des zentralen Voxels 107-C zu verbessern, wird der MR-Fingerabdruck des zentralen Voxels 107-C und seiner räumlichen Nachbarn 107-N verarbeitet. Der räumliche Kontext kann ebenfalls zur Schätzung von Fehlerwahrscheinlichkeiten der Berechnungen herangezogen werden.

In Schritt S402 werden anschließend die MR-Parameter für das zentrale Voxel 107-C durch das Netz 100 ausgegeben. Durch Verarbeiten eines MR-Fingerabdrucks eines Voxels 107 können die MR-Parameter für dessen räumliche Nachbarn ebenfalls vorhergesagt werden.

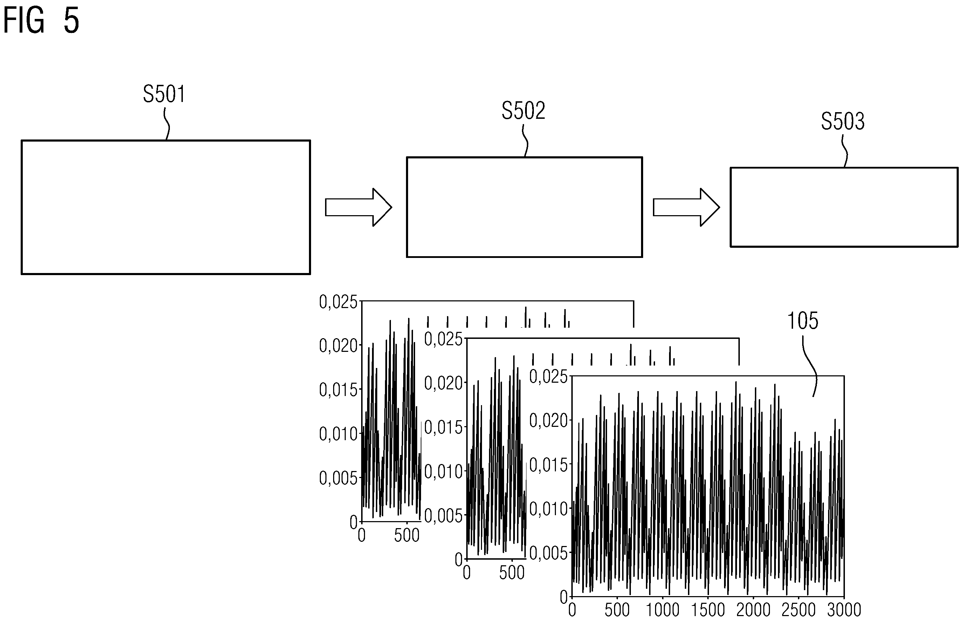

Fig. 5 zeigt ein Blockdiagramm zum Erzeugen einer Aufnahmesequenz, Messen von Zeitserien und Trainieren des neuronalen Netzes. In Schritt S501 wird eine Aufnahmevorschrift erzeugt, wie beispielsweise eine Pulssequenz. In Schritt S502 wird die Aufnahmevorschrift verwendet, um den MR-Fingerabdruck des Voxels 107 zu messen. In Schritt S503 wird das Netz 100 mit dem gemessenen MR-Fingerabdruck trainiert.
Die Gestaltung des Netzes 100 kann mit der Lehre, d.h. dem neuronalen Netz der Patentanmeldung US 14/682,220 verknüpft werden. Das Netz der Patentanmeldung US 14/682,220 kann dann verwendet werden, um eine ideale Aufnahmesequenz zu erzeugen, die einen bestmöglichen Kontrast zwischen zwei MR-Fingerabdrücken erzeugt. Diese Fingerabdrücke können für ein Training des Netzes 100 zum Vorhersagen von MR-Parametern verwendet werden.

Die Kombination eines MR-Fingerabdruckverfahrens mit tiefgehenden Lernverfahren ermöglicht eine direkte Vorhersage von quantitativen Karten (Maps), wie beispielsweise T1- und T2-Relaxationszeiten, mithilfe eines trainierten neuronalen Netzes aus dem erfassten MR-Fingerabdruck der jeweiligen Voxel. Dahingegen beruht der bekannte Schritt eines Abgleichens eines MR-Fingerabdrucks auf einem Abgleichen einer gemessenen Zeitserie mit einem Verzeichnis simulierter Zeitserien.

Das Verfahren und/oder das neuronale Netz kann durch ein digitales Computerprogramm mit Programmabschnitten zum Durchführen der Verfahrensschritte implementiert sein, wenn das Computerprogramm auf einem Computer ausgeführt wird. Der Computer umfasst dazu einen computer-lesbaren Speicher zum Speichern des Computerprogramms und einen Prozessor zum Abarbeiten des Computerprogramms. Das Computerprogramm kann wiederrum auf einem externen Datenspeicher gespeichert sein, von dem das Computerprogramm in den internen Datenspeicher des Computers geladen werden kann. Der externe Datenspeicher ist beispielsweise eine CD-ROM, ein USB-Flash-Speicher oder ein Speicherlaufwerk im Internet.

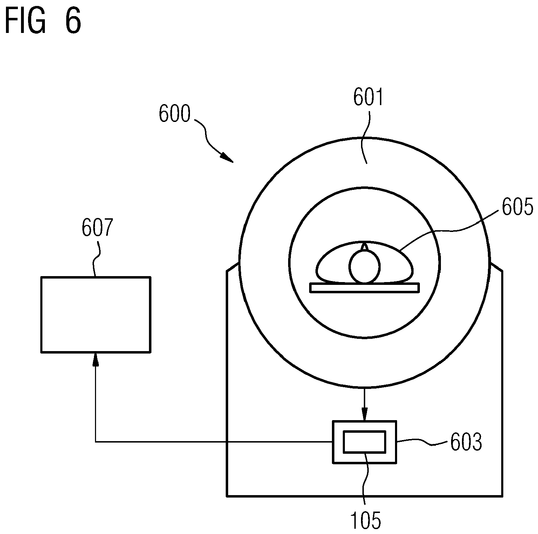

Fig. 6 zeigt schematisch ein Magnetresonanzgerät 600 zum Ermitteln von MR-Parametern auf Basis von MR-Fingerabdrücken 105. Das Magnetresonanzgerät 600 umfasst eine Erfassungseinheit 601 zum Erfassen des MR-Fingerabdrucks 105 zumindest eines Voxels 107 eines Untersuchungsobjektes mittels einer Pulssequenz, beispielsweise eines Patienten 605. In einem Beispiel umfasst die Erfassungseinheit 601 einen Magneten zum Anlegen eines Magnetfeldes und eine Steuerschaltung zum Anlegen von RF-Pulsen und Gradientenfeldern. Die Erfassungseinheit 601 kann ferner eine Empfängereinheit aufweisen, zum Beispiel eine Spule, die die Magnetisierung der angeregten Kernspins durch Ströme erfasst, die in der Spule induziert werden.

Zusätzlich umfasst das Magnetresonanzgerät 600 eine Bestimmungseinheit 603 zum Bestimmen des MR-Parameters durch Eingeben des gemessenen MR-Fingerabdrucks 105 in die Eingabeschicht 101-1 des trainierten neuronalen Netzes 100 und durch Ausgeben des zumindest einen MR-Parameters zu dem MR-Fingerabdruck 105 des Voxels 107 an der Ausgabeschicht 101-O des trainierten neuronalen Netzes 100. Dadurch lassen sich jedem Voxel auf schnelle und einfache Weise entsprechende MR-Paramater zuordnen und Schnittbilder des Patienten, z.B. in einer medizinischen Untersuchung, gewinnen. Die entsprechenden Karten der ermittelten MR-Parameter können auf einem Bildschirm 607 angezeigt werden.

Die Bestimmungseinheit 603 kann durch ein Computermodul mit einem digitalen Speicher und einem Prozessor ausgebildet sein, das in der Lage ist, ein Computerprogramm zur Bestimmung des MR-Parameters auszuführen. Die Bestimmungseinheit 603 kann aber auch durch eine entsprechend ausgebildete elektronische Schaltung implementiert sein.

Durch das Magnetresonanzgerät 600 und das Verfahren auf Basis des neuronalen Netzes 100 lassen sich die folgenden Vorteile erreichen:
1) Eine Geschwindigkeit der Quantifizierung wird erhöht. Sobald das künstliche neuronale Netz 100 angelernt ist, wird eine schnelle Berechnung von MR-Parametern ermöglicht, die ein Zuordnen von quantitativen MR-Parametern zu entsprechenden Voxeln 107 im Vergleich zu den weiter oben beschriebenen Matching-Verfahren beschleunigt.
2) Zudem kann der räumliche Kontext eines Voxels 107 berücksichtigt werden. Das künstliche neuronale Netz 100 kann trainiert werden, um nicht nur die MR-Parameter eines einzigen Voxels 107 vorherzusagen, sondern ebenso für die räumliche Umgebung des Voxels 107-1, ..., 107-9. Die räumliche Umgebung eines einzigen Voxels 107-C kann verwendet werden, um eine stabilere Schätzung oder eine Fehlerabschätzung zu erhalten.
3) Nachdem das künstliche neuronale Netz 100 trainiert worden ist, wird kein Verzeichnis mehr für den Schritt einer Quantifizierung der MR-Parameter benötigt. Das Verzeichnis wird durch das neuronale Netz 100 ersetzt, welches aber weniger Speicherplatz benötigt und dessen Speicherplatzbedarf nicht von der Anzahl möglicher Parameterkombinationen abhängig ist. Das künstliche neuronale Netz 100 kann ebenfalls jede Kombination an MR-Parametern vorhersagen, wohingegen ein Verzeichnis lediglich eine begrenzte Auflösung aufweist, die zu hohen Fehlern führt (siehe beispielsweise Wang Z, Zhang Q, Yuan J, Wang X, "MRF Denoising with Compressed Sensing and Adaptive Filtering", Biomedical Imaging (ISBI), 2014 IEEE 11th International Symposium on. IEEE, 2014). In gleicher Weise wachsen die Speicheranforderung, die Simulationszeit des Verzeichnisses und die Zeit, die für das Abgleichen mittels Mustererkennung erforderlich ist, in Abhängigkeit der Auflösung des Verzeichnisses. Diese Probleme werden durch ein künstliches neuronales Netz 100 gelöst, das kein Verzeichnis und Muster-Abgleichverfahren für die Berechnung der quantitativen Parameter eines Voxels 107 verwendet.

Alle in Verbindung mit einzelnen Ausführungsformen der Erfindung erläuterten und gezeigten Merkmale können in unterschiedlicher Kombination in dem erfindungsgemäßen Gegenstand vorgesehen sein, um gleichzeitig deren vorteilhafte Wirkungen zu realisieren.

Alle Verfahrensschritte können durch Vorrichtungen implementiert werden, die zum Ausführen des jeweiligen Verfahrensschrittes geeignet sind. Alle Funktionen, die von gegenständlichen Merkmalen ausgeführt werden, können ein Verfahrensschritt eines Verfahrens sein.

Der Schutzbereich der vorliegenden Erfindung ist durch die Ansprüche gegeben und wird durch die in der Beschreibung erläuterten oder den Figuren gezeigten Merkmale nicht beschränkt.

## Patentansprüche

1. Verfahren zum Ermitteln von MR-Parametern (T1, T2), mit den Schritten:
- Erfassen (S101) eines MR-Fingerabdrucks (105) eines Voxels (107) mittels einer Pulssequenz;
- Eingeben (S102) des MR-Fingerabdrucks (105) in die Eingabeschicht (101-1) eines trainierten neuronalen Netzes (100); und
- Ausgeben (S103) zumindest eines MR-Parameters (T1, T2) zu dem MR-Fingerabdruck (105) des Voxels (107) an der Ausgabeschicht (101-O) des trainierten neuronalen Netzes (100).

2. Verfahren nach Anspruch 1, wobei die Pulssequenz eine MRF-FISP-Pulssequenz ist.

3. Verfahren nach einem der vorangehenden Ansprüche, wobei der zumindest eine MR-Parameter (T1, T2) eine T1-Relaxationszeit und/oder eine T2-Relaxationszeit umfasst.

4. Verfahren nach einem der vorangehenden Ansprüche, wobei mehrere MR-Fingerabdrücke (105) benachbarter Voxel (107-C, 107-N) in die Eingabeschicht (101-1) eingegeben werden und zumindest ein gemeinsamer MR-Parameter (T1, T2) für die mehreren MR-Fingerabdrücke (105) an der Ausgabeschicht (101-O) ausgegeben wird.

5. Verfahren nach einem der Ansprüche 1 bis 3, wobei ein einziger MR-Fingerabdruck (105) eines Voxels (107) in die Eingabeschicht (101-1) eingegeben wird und mehrere MR-Parameter (T1, T2) für benachbarte Voxel (107-1, ..., 107-9) an der Ausgabeschicht (101-O) ausgegeben werden.

6. Verfahren nach einem der vorangehenden Ansprüche, wobei ein Kontrast zwischen zwei MR-Fingerabdrücken (105) mittels des neuronalen Netzes (100) ausgewertet wird.

7. Verfahren nach einem der vorangehenden Ansprüche, wobei das Verfahren für eine Vielzahl benachbarter Voxel (107) angewendet wird.

8. Computerprogramm mit Programmabschnitten zum Durchführen der Verfahrensschritte nach einem der Ansprüche 1 bis 7, wenn das Computerprogramm auf einem Computer ausgeführt wird.

9. Speicher mit einem Computerprogramm nach Anspruch 8.

10. Neuronales Netz (100), mit:
- einer Eingabeschicht (101-1) zum Eingeben eines MR-Fingerabdrucks (105) eines Voxels (107);
- mehreren verdeckten Schichten (101-H) zum Verarbeiten des MR-Fingerabdrucks (105); und
- einer Ausgabeschicht (101-O) zum Ausgeben zumindest eines MR-Parameters (T1, T2) zu dem MR-Fingerabdruck (105) des Voxels (107).

11. Magnetresonanzgerät (600) zum Ermitteln von MR-Parametern (T1, T2), mit:
- einer Erfassungseinheit (601) zum Erfassen eines MR-Fingerabdrucks (105) eines Voxels (107) mittels einer Pulssequenz; und
- einer Bestimmungseinheit (603) zum Bestimmen zumindest eines MR-Parameters (T1, T2) durch Eingeben des MR-Fingerabdrucks (105) in die Eingabeschicht (101-1) eines trainierten neuronalen Netzes (100); und Ausgeben des zumindest einen MR-Parameters (T1, T2) zu dem MR-Fingerabdruck (105) des Voxels (107) an der Ausgabeschicht (101-O) des trainierten neuronalen Netzes (100).

12. Magnetresonanzgerät (600) nach Anspruch 11, wobei als das trainierte neuronale Netz in der Bestimmungseinheit (603) ein neuronales Netz nach Anspruch 10 vorgesehen ist, das trainiert ist.

13. Magnetresonanzgerät (600) nach Anspruch 11 oder 12, wobei das Magnetresonanzgerät (600) ausgebildet ist, MR-Parameter (T1, T2) einer Vielzahl benachbarter Voxel (107) zu ermitteln.

## Geänderte Patentansprüche

### Geänderte Patentansprüche gemäss Regel 137(2) EPÜ.

1. Verfahren zum Ermitteln von MR-Parametern (T1, T2), mit den Schritten:
- Erfassen (S101) eines MR-Fingerabdrucks (105) eines Voxels (107) mittels einer Pulssequenz;
- Eingeben (S102) des MR-Fingerabdrucks (105) in die Eingabeschicht (101-I) eines trainierten neuronalen Netzes (100); und
- Ausgeben (S103) zumindest eines MR-Parameters (T1, T2) zu dem MR-Fingerabdruck (105) des Voxels (107) an der Ausgabeschicht (101-O) des trainierten neuronalen Netzes (100), wobei der zumindest eine MR-Parameter (T1, T2) eine T1-Relaxationszeit und/oder eine T2-Relaxationszeit umfasst.

2. Verfahren nach Anspruch 1, wobei die Pulssequenz eine MRF-FISP-Pulssequenz ist.

3. Verfahren nach einem der vorangehenden Ansprüche, wobei mehrere MR-Fingerabdrücke (105) benachbarter Voxel (107-C, 107-N) in die Eingabeschicht (101-I) eingegeben werden und zumindest ein gemeinsamer MR-Parameter (T1, T2) für die mehreren MR-Fingerabdrücke (105) an der Ausgabeschicht (101-O) ausgegeben wird.

4. Verfahren nach einem der Ansprüche 1 bis 2, wobei ein einziger MR-Fingerabdruck (105) eines Voxels (107) in die Eingabeschicht (101-I) eingegeben wird und mehrere MR-Parameter (T1, T2) für benachbarte Voxel (107-1, ..., 107-9) an der Ausgabeschicht (101-O) ausgegeben werden.

5. Verfahren nach einem der vorangehenden Ansprüche, wobei ein Kontrast zwischen zwei MR-Fingerabdrücken (105) mittels des neuronalen Netzes (100) ausgewertet wird.

6. Verfahren nach einem der vorangehenden Ansprüche, wobei das Verfahren für eine Vielzahl benachbarter Voxel (107) angewendet wird.

7. Computerprogramm mit Programmabschnitten zum Durchführen der Verfahrensschritte nach einem der Ansprüche 1 bis 6, wenn das Computerprogramm auf einem Computer ausgeführt wird.

8. Speicher mit einem Computerprogramm nach Anspruch 7.

9. Neuronales Netz (100), mit:
- einer Eingabeschicht (101-I) zum Eingeben eines MR-Fingerabdrucks (105) eines Voxels (107);
- mehreren verdeckten Schichten (101-H) zum Verarbeiten des MR-Fingerabdrucks (105); und
- einer Ausgabeschicht (101-O) zum Ausgeben zumindest eines MR-Parameters (T1, T2) zu dem MR-Fingerabdruck (105) des Voxels (107), wobei der zumindest eine MR-Parameter (T1, T2) eine T1-Relaxationszeit und/oder eine T2-Relaxationszeit umfasst.

10. Magnetresonanzgerät (600) zum Ermitteln von MR-Parametern (T1, T2), mit:
- einer Erfassungseinheit (601) zum Erfassen eines MR-Fingerabdrucks (105) eines Voxels (107) mittels einer Pulssequenz; und
- einer Bestimmungseinheit (603) zum Bestimmen zumindest eines MR-Parameters (T1, T2) durch Eingeben des MR-Fingerabdrucks (105) in die Eingabeschicht (101-I) eines trainierten neuronalen Netzes (100); und Ausgeben des zumindest einen MR-Parameters (T1, T2) zu dem MR-Fingerabdruck (105) des Voxels (107) an der Ausgabeschicht (101-O) des trainierten neuronalen Netzes (100), wobei der zumindest eine MR-Parameter (T1, T2) eine T1-Relaxationszeit und/oder eine T2-Relaxationszeit umfasst.

11. Magnetresonanzgerät (600) nach Anspruch 10, wobei als das trainierte neuronale Netz in der Bestimmungseinheit (603) ein neuronales Netz nach Anspruch 10 vorgesehen ist, das trainiert ist.

12. Magnetresonanzgerät (600) nach Anspruch 10 oder 11, wobei das Magnetresonanzgerät (600) ausgebildet ist, MR-Parameter (T1, T2) einer Vielzahl benachbarter Voxel (107) zu ermitteln.
